# EUROPEAN PATENT SPECIFICATION

(11) **EP 1 974 904 B1**
(45) Date of publication and mention of the grant of the patent: **26.12.2012**
(21) Application number: 08153584.1
(22) Date of filing: 28.03.2008
(51) Int. Cl.: B32B 5/00, A61L 9/16, B01D 39/14, B01D 53/04

(54) **Functional particle carrying sheet and manufacturing method thereof**
Funktionelles, von einem Partikel getragenes Blatt und Herstellungsverfahren dafür
Feuille portant des particules fonctionnelles et son procédé de fabrication

(30) Priority: 29.03.2007 JP 2007088725
(43) Date of publication of application: 01.10.2008
(73) Proprietor: JAPAN VILENE COMPANY, LTD., Tokyo 104-8423 (JP)
(72) Inventor: Aikawa, Toshio c/o Japan Vilene Company , Ltd., Ibaraki 306-0213 (JP); Tanno, Tomoaki c/o Japan Vilene Company, Ltd., Ibaraki 306-0213 (JP); Matsui, Yasuhiro c/o Japan Vilene Company, Ltd., Ibaraki 306-0213 (JP); Kobori, Satoru c/o Japan Vilene Company, Ltd., Ibaraki 306-0213 (JP)
(74) Representative: Viering, Jentschura & Partner

(56) References cited:
- DATABASE WPI Week 200432 Thomson Scientific, London, GB; AN 2004-344025 XP002502784 & JP 2004 113433 A (JAPAN VILENE CO LTD) 15 April 2004 (2004-04-15)
- DATABASE WPI Week 200122 Thomson Scientific, London, GB; AN 2001-215526 XP002506767 & JP 2001 038116 A (TOYO ROKI SEIZO KK) 13 February 2001 (2001-02-13)
- DATABASE WPI Week 200376 Thomson Scientific, London, GB; AN 2003-807040 XP002502782 & JP 2003 079715 A (JAPAN VILENE CO LTD) 18 March 2003 (2003-03-18)

## Description

### Technical Field

The present invention relates to a sheet in which a functional particle brings out a beneficial function which can be preferably used particularly as a filter material for removing a harmful material from a gas or a liquid by a function, for example, a deodorant function, a catalyst function or the like, such as a deodorant filter material used by being installed in an air conditioning device in a living environment for filtering and cleaning a fluid contaminated by an odor component, a filter material for a chemical filter removing a gaseous contaminant included in the air or the ambient atmosphere in a production facility of a semiconductor and a liquid crystal, a clean room or the like.

### Background Art

Conventionally, for removing the uncomfortable odor material in the living environment and removing the gaseous contaminant included in the air or the ambient atmosphere in the production facility of the semiconductor and the liquid crystal, the clean room or the like, various deodorant filter material, the filter material for the chemical filter and the like have been proposed. As the deodorant filter material and the filter material for the chemical filter, has been known, for example, a structure which retaining a gas removing particle constituted by an activated carbon, various chemical absorbent, an ion exchange resin or a catalyst on a breathable sheet-like material, a structure which couples the gas removing particles by a thermoplastic resin so as to form a porous material, or a structure which charges the gas, removing particles in a breathable case.

In a technique obtained by improving the conventional technique, carrying the gas removing particle as mentioned above, for example, patent document 1 describes a laminated type deodorant filter material in which deodorant particles constructing a lamination unit are firmly attached to each other via a web constituted by a hot melt resin. In this laminated type deodorant filter material, a structure is made such that a layer having a height of one particle is laminated so as to form two layers, and the individual particles are firmly attached to each other. Accordingly, despite that the deodorant function of the particle has increased, there can be obtained an effect that a pressure loss can be reduced without obstructing the original deodorant function of the particle. However, if a filter element is formed by pleating the filter materials mentioned above in a zigzag shape, there is generated a problem that a sharp shape is not obtained in a peak portion or a trough portion of the pleat shape due to the two layers, or the particle jumps out of a base material mounting the particle thereon.

In order to prevent the particle from existing in a portion in which the peak portion or the trough portion of the pleat shape is formed, there has been considered to apply an adhesive agent to the other portions except the linear portion corresponding to the peak portion and the trough portion on the breathable base material so as to attach the functional particle via the adhesive agent. A bactericidal sheet in accordance with a similar method thereto is described in patent document 2. However, since the bactericidal sheet corresponds to a bactericidal sheet structured such that a granular bactericidal substance is attached to an adhesive agent applied like a dotted shape, a linear shape or a net shape to a fiber base fabric, and the functional particle serving as the bactericidal substance is laminated only in one layer, it is impossible to increase the performance of the functional particle.

Furthermore, in the other technique, patent document 3 describes a filter structured such that the functional material is dispersed in a sheet-like filter material, and the filter in which a ventilation resistance is changed by changing the distribution density of the function material with respect to the filter material. However, since the filter is structured such that the function material is dispersed in the sheet-like filter material, and the individual function materials are not particularly fixed, for example, by the adhesive agent or the like, there is a problem that the function materials come into collision with each other so as to be broken due to an oscillation applied during the use of the filter material or the like, and fragments or fine particles fly in all directions. Furthermore, since the particles come into contact with each other and overlap in several layers in the case that the function material is constituted by the particle, the layer with the height of one particle is not laminated so as to form two layers as is different from the laminated type deodorant filter material in the patent document 1. Accordingly, there is a problem that it is impossible to obtain an effect of reducing the pressure loss as well as an increase of the deodorant function of the particle.

Accordingly, the applicant of the present invention has proposed a functional particle carrying sheet described in patent document 4. The functional particle carrying sheet is structured such as to have two particle firmly attaching sheets in which a functional particle is firmly attached onto a surface of a breathable base material via a first adhesive agent applied to the portion except to the band-like space portions provided at a fixed interval, and structured such that the functional particle in one particle firmly attaching sheet and the functional particle in the other particle firmly attaching sheet are bonded via a second adhesive agent in such a manner that a position of the space portion in one particle firmly attaching sheet comes to an intermediate position of two positions of the space portion in the other particle firmly attaching sheet.

However, there is a problem that it is hard to arrange such that the position of the space portion in one particle firmly attaching sheet comes to the intermediate position of the two positions of the space portion in the other particle firmly attaching sheet, at the time of manufacturing the functional particle carrying sheet. For example, since some elongation is generated during the manufacture of the particle firmly attaching sheet, the intervals of the space portions of two particle firmly attaching sheets having different manufacturing times are subtly differentiated. Accordingly, there is a problem that the filter element having an irregular height in the fold peak is formed by pleating two particle firmly attaching sheets in a zigzag shape after bonding them so as to form the filter element. Furthermore, for example, it is necessary to make speeds for feeding out two functional particle carrying sheets identically, and it is necessary to position it such that the position of the space portion in one particle firmly attaching sheet comes to the intermediate position of the two positions of the space portion in the other particle firmly attaching sheet. Therefore, there is a problem that a manufacturing apparatus is demanded so as to have a high technique, and as a result, a manufacturing cost becomes high.
Patent Document 1: Japanese Unexamined Patent Publication No. 11-57467
Patent Document 2: Japanese Utility Model No. 3032523
Patent Document 3: Japanese Unexamined Patent

DATABASE WPI Week 200122 Thomson Scientific, London, GB; AN 2001-215526 XP002506767 & JP 2001 038116 A (TOYO ROKI SEIZO KK) 13 February 2001 (2001-02-13) discloses a filter containing activated carbon functional material distributed in a sheet-like filter medium. The distribution density of the functional material is varied for imparting air passage resistance.

DATABASE WPI Week 200376 Thomson Scientific, London, GB; AN 2003-807040 XP002502782 & JP 2003 079715 A (JAPAN VILENE CO LTD) 18 March 2003 (2003-03-18) discloses a filter medium comprising a laminate of air permeable cover material and a gas removal material having adhesive on at least one surface. The gas removal material holds the gas removal particles.

### Disclosure of the Invention

### Problem to be Solved by the Invention

An object of the present invention is to provide a functional particle carrying sheet which can solve the problem mentioned above, reduce pressure loss at the time when a fluid passes, without obstructing an original function of the functional particle despite that a function of the functional particle has increased, is constituted by a particle layer having a two-layer structure, which can form a sharp shape by a peak portion and a trough portion of a pleat shape in the case that the sheet is pleated in a zigzag shape, for example, in forming a filter element, it can prevent the functional particle from jumping out from the base material carrying the functional particle, which can form a filter element having a precisely aligned height of a fold peak and good quality, is easily manufactured and can reduce the manufacturing cost, the manufacturing method of the functional particle carrying sheet, and the functional particle carrying element using the functional particle carrying sheet.

### Means for Solving the Problem

The invention provides a functional particle carrying sheet and a manufacturing method of a functional particle carrying sheet according to the independent claims. Further embodiments of the invention are described in the dependent claims.

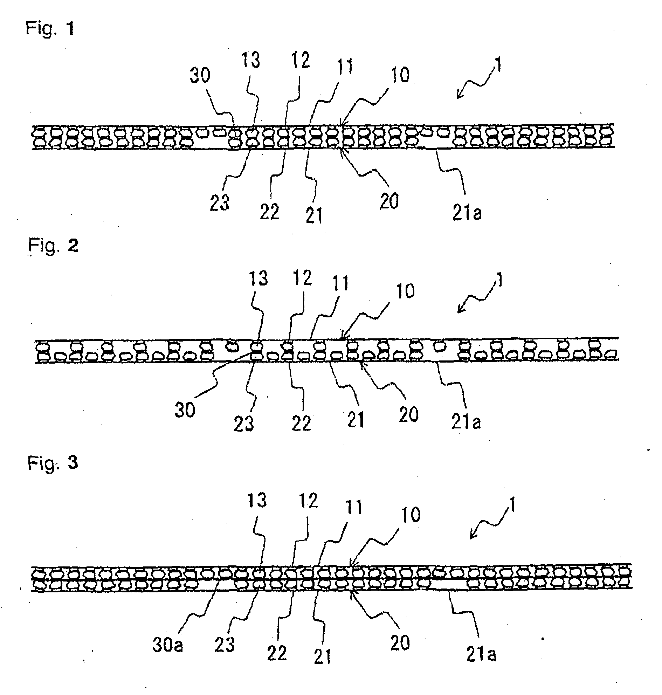

In accordance with the first aspect of the present invention, as illustrated in Fig. 1, there is provided a functional particle carrying sheet comprising:
a first particle firmly attaching sheet 10 structured such that the functional particle 13 is firmly attached onto the surface of a first breathable base material 11 via a first adhesive agent 12; and
a second particle firmly attaching sheet 20 structured such that a functional particle 23 is firmly attached to a portion except the band-like space portions 21a provided at a fixed interval on the surface of a second breathable base material 21, via a second adhesive agent 22, wherein the functional particle 13 in the first particle firmly attaching sheet 10 and the functional particle 23 in the second particle firmly attaching sheet 20 are bonded via a third adhesive agent 30. In accordance with the invention on the basis of the first aspect, the functional particle carrying sheet corresponds to the sheet constituted by the particle layer having a two-layer structure, which can reduce the pressure loss at a time when the fluid passes without obstructing the original function of the functional particle despite that the function of the functional particle has increased, which can form a sharp shape by a peak portion and a trough portion of the pleat shape in the case that the sheet is pleated in a zigzag shape, for example, in forming a filter element, which can prevent the functional particle from jumping out from the base material carrying the functional particle, and can form a filter element having a precisely al igned height of the fold peaks and good quality. Furthermore, in the manufacturing of the functional particle carrying sheet, an advantage is obtained that an apparatus demanding a high technique is not necessary, but it is also easy to manufacture and the manufacturing cost can be reduced, such that the manufacturing can be achieved by a simple pleating machine.

In accordance with the second aspect of the present invention, the width of the band-like space portion can be between 0.5 and 10 mm. Since the width of the band-like space portion is between 0.5 and 10 mm, the advantage mentioned above becomes more significant.

In accordance with the third aspect of the present invention, an average particle diameter of the functional particle can be between 0.147 and 1.65 mm. Since the average particle diameter of the functional particle is between 0.147 and 1.65 mm, the advantage mentioned above becomes more significant.

In accordance with the fourth aspect of the present invention, the functional particle can be firmly attached at 20 to 99 % of a maximum firmly attaching amount onto a surface of the first breathable base material 11. Since the functional particle is firmly attached at 20 to 99 % of the maximum firmly attaching amount, the advantage mentioned above becomes more significant.

In accordance with the fifth aspect of the present invention, the interval between the functional particles firmly attached to the first particle firmly attaching sheet can be greater than the interval between the functional particles firmly attached to the second particle firmly attaching sheet. In accordance with the structure mentioned above, an advantage can be obtained that a sharper shape can be obtained in the peak portion and the trough portion of the pleated shape while sufficiently achieving the function of the functional particle, and the advantage mentioned above becomes more significant.

In accordance with the sixth aspect of the present invention, the rate at which the functional particle is firmly attached onto the surface of the first breathable base material with respect to the maximum firmly attaching amount can be less than the rate at which the functional particle is firmly attached onto the surface of the portion except the band-like space portion of the second breathable base material with respect to the maximum firmly attaching amount. In accordance with the structure mentioned above, an advantage can be obtained that a sharper shape can be obtained in the peak portion and the trough portion of the pleated shape while sufficiently achieving the function of the functional particle, and the advantage mentioned above becomes more significant.

In accordance with the seventh aspect of the present invention, the bonding via the third adhesive agent 30 can be achieved by an application of a hot melt resin. Accordingly, it is possible to prevent the function of the functional particle from being greatly damaged and furthermore it is possible to reduce the reduction of the pressure loss.

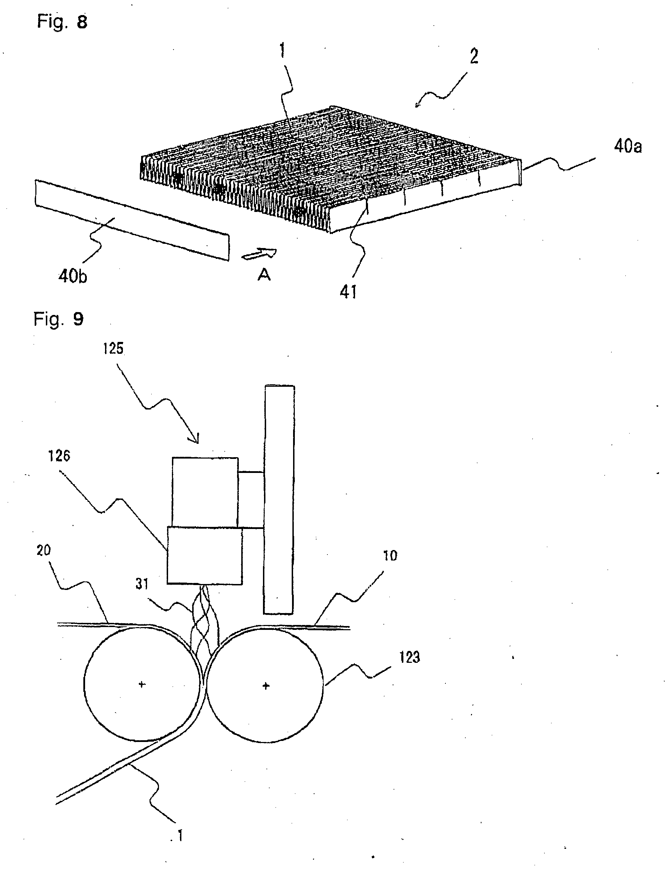

In accordance with the eighth aspect of the present invention, as exemplified in Fig. 8, a functional particle carrying element 2 is provided, wherein the functional particle carrying sheet as recited above is pleated, and frame materials 40a and 40b are firmly attached to the peripheral edge portion of the functional particle carrying sheet 1. The functional particle carrying element 2 can have a sharp shape by a peak portion and a trough portion of a pleated shape, and has an advantage that the functional particle does not jump out of the base material carrying the functional particle, and that it is easy to manufacture the element.

In accordance with the ninth aspect of the present invention, a manufacturing method of the functional particle carrying sheet is provided, comprising the following steps :
preparing the first particle firmly attaching sheet 10 structured such that the functional particle 13 is firmly attached onto the surface of a first breathable base material 11 via the first adhesive agent 12, and the second particle firmly attaching sheet 20 structured such that the functional particle 23 is firmly attached to the portions except the band-like space portions 21a provided at a fixed interval on the surface of a second breathable base material 21 via the second adhesive agent 2; and
next bonding the functional particle 13 in the first particle firmly attaching sheet 10 and the functional particle 23 in the second particle firmly attaching sheet 20 via the third adhesive agent 30.

In accordance with the tenth aspect of the present invention, the method of bonding via the third adhesive agent 30 can correspond to a method of simultaneously applying a hot melt resin constituted by the third adhesive agent 30 to the surface of the functional particle in the first particle firmly attaching sheet 10 and a surface of the functional particle in the second particle firmly attaching sheet 20, and next overlapping and bonding the first particle firmly attaching sheet 10 and the second particle firmly attaching sheet 20. Since it is possible to firmly bond the functional particles even if an applying amount of the third adhesive agent is small, an advantage is obtained that it is possible to manufacture the functional particle carrying sheet having a reduced pressure loss. Furthermore, an advantage is obtained that it is possible to reduce a cost.

### Effect of the Invention

In accordance with the present invention, the sheet constituted by the two-layer structured particle layer can be obtained, which can reduce the pressure loss at the time when the fluid passes, without obstructing the original function of the functional particle, despite that the function of the functional particle has increased. Furthermore, in the case that the sheet is pleated in the zigzag shape, for example, so as to be formed as the filter element, the advantage is obtained that it is possible to obtain the sharp shape by the peak portion and the trough portion of the pleat shape, and that the functional particle does not jump out of the base material carrying the functional particle, and the filter element is formed such that the heights of the fold peaks are precisely aligned and have good quality. Furthermore, in the manufacturing of the functional particle carrying sheet, not only the apparatus demanding the high technique is not necessary, but also the simple pleating machine that can achieve the method, whereby it is easy to manufacture the functional particle carrying sheet. Accordingly, it is possible to provide the functional particle carrying sheet and the manufacturing method thereof which can reduce the manufacturing cost, and the functional particle carrying element using the functional particle carrying sheet.

### Brief Description of the Drawings

Fig. 1 is a cross sectional schematic view showing one embodiment of a functional particle carrying sheet in accordance with the present invention;
Fig. 2 is a cross sectional schematic view showing the other embodiment of the functional particle carrying sheet in accordance with the present invention;
Fig. 3 is a cross sectional schematic view showing the other embodiment of the functional particle carrying sheet in accordance with the present invention;
Fig. 4 is a schematic plan view showing one embodiment of a first particle firmly attaching sheet;
Fig. 5 is a schematic plan view showing one embodiment of a second particle firmly attaching sheet;
Fig. 6 is a cross sectional schematic view showing one embodiment of the first particle firmly attaching sheet (or the second particle firmly attaching sheet);
Fig. 7 is a cross sectional schematic view showing one embodiment a pleating work of the functional particle carrying sheet in accordance with the present invention;
Fig. 8 is a perspective view showing one embodiment of a functional particle carrying element in accordance with the present invention, and is a view exemplifying an aspect in which a frame material is installed in a direction of an arrow A;
Fig. 9 is a view showing one embodiment of an apparatus which is preferably used for manufacturing the functional particle carrying sheet in accordance with the present invention; and
Fig. 10 is a view showing one embodiment of the apparatus which is preferably used for manufacturing of the functional particle carrying sheet in accordance with the present invention.

### Best Mode for Carrying Out the Invention

A detailed description given below of preferable embodiments of the functional particle carrying sheet and the manufacturing method thereof in accordance with the present invention, and a functional particle carrying element using the functional particle carrying sheet. In this case, the manufacturing method of the functional particle carrying sheet is explained in the description of the functional particle carrying sheet in accordance with the present invention.

The functional particle carrying sheet in accordance with the present invention is constituted by a functional particle carrying sheet 1 structured such as to have a first particle firmly attaching sheet 10 in which the functional particle 13 is firmly attached onto the surface of the first breathable base material 11 via the first adhesive agent 12, and the second particle firmly attaching sheet 20 in which the functional particle 23 is firmly attached to the portions except the band-like space portions 21a which are provided at a fixed interval on the surface of the second breathable base material 21 via the second adhesive agent 22, and structured such that the functional particle 13 in the first particle firmly attaching sheet 10 and the functional particle 23 in the second particle firmly attaching sheet 20 are bonded via a third adhesive agent 30, as exemplified in Fig. 1.

The first breathable base material 11 or the second breathable base material 21 (hereinafter, the first breathable base material and the second breathable base material are collectively referred simply to as the "breathable base material") is not particularly limited, as far as it is a sheet-like material having breathability. For example, a porous material such as a nonwoven fabric, a woven fabric, a membrane, a filter paper, a sponge or the like can be listed up, and the nonwoven fabric is preferable because the nonwoven fabric can increase the breathability. Furthermore, if the sheet-like material used for the breathable base material is made of a high polymer material, the following capability of the pleating fold work in the filter work or the like is high and the durability is excellent Accordingly, the sheet-like material made of the high polymer material can be preferably used. In the nonwoven fabric mentioned above, a nonwoven fabric can be listed up, obtained by a dry process method coupling the fibers in accordance with an adhesion, an entangling or the like after forming a staple fiber into the fiber web by using a carding machine or the like, a nonwoven fabric constituted by a continuous fiber in accordance with a spun bond method, a nonwoven fabric obtained by a wet process method or the like. In the breathable base material mentioned above, it is preferable that the mass per unit area is between 10 and 200 g/m² , and it is further preferable that the mass per unit area be between 10 and 50 g/m². Furthermore, it is preferable that the pressure loss is at a face velocity of 0.5 m/sec, equal to or less than 100 Pa. Furthermore, it is further preferable that the pressure loss is equal to or less than 50 Pa.

Furthermore, the functional particle is not particularly limited as far as the functional particle is constituted by a solid particle which can be firmly attached to the surface of the breathable base material, and can achieve the function with respect to a gas or a liquid passing through the breathable base material, but may be constituted by an inorganic substance or an organic substance, and it is possible to appropriately select one or more kinds of the solid particle mentioned above. Furthermore, the functional particle applied to the first breathable base material and the functional particle applied to the second breathable base material may be identical or different. In the functional particle mentioned above, for example, a solid particle can be listed up having the functionalities such as a deodorant, a gas removal, a catalyst, a photocatalyst, a water absorption, an ion exchange, an electromagnetic wave radiation, aniongenerator, an antimicrobial action, a flame resister, an electromagnetic wave shield, a sound insulator, and the like. As a material of the solid particle, various materials can be listed up, for example, an activated carbon, a zeolite, a titanium oxide, a water absorbing resin, an ion exchanging resin, a metal particle, a tourmaline, a calcium carbonate, and the like.

As the solid particle having the gas removing function capable of absorbing a gaseous material or changing to a material tending to absorb the gaseous material, and used for removing the uncomfortable odor material in the living environment or removing the air or the gaseous contaminant included in the ambient atmosphere in the production facility of the semiconductor or the liquid crystal, the clean room or the like, in the functional particle, there are, for example, the activated carbon, an impregnated carbon obtained by adding various chemical components capable of removing an acid gas or a basic gas, the zeolite, various chemical absorbents, the ion exchange resin, the catalyst such as the photocatalyst or the like, and the like. It is possible to appropriately select one, two or more from them. Furthermore, for example, in the case that the activated carbon is selected for the purpose of deodorant from them, it is preferable to employ a porous material in which a specific surface area is equal to or more than 200 m²/g, it is more preferable that the specific surface area is equal to or more than 500 m² /g, and it is further preferable that the specific surface area is equal to or more than 800 m² /g. Furthermore, the particle diameter of the gas removing particle for the purpose of deodorant is preferably set such that an average particle diameter is set to be equal to or more than 0.147 mm (100 mesh) and equal to or less than 1.65 mm (10 mesh) for achieving a high efficiency and a low pressure loss. Furthermore, it is more preferable to set the average particle diameter between 0.212 mm (70 mesh) and 1.0 mm (16 mesh). If the gas removing particle having an average particle diameter which is smaller than the lower limit of the preferable range is used, an initial gas removing efficiency can be set to high, however, there is a case that the pressure loss becomes large. In this case, the average particle diameter in the case that the particle diameter of the gas removing particle has a distribution is expressed by a mass average value of each of the particle diameters.

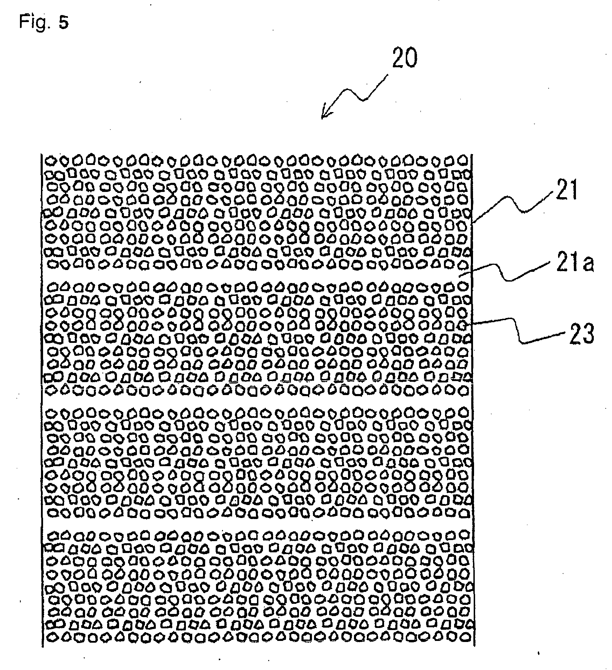

In the present invention, the second particle firmly attaching sheet 20 is structured, as shown in Figs. 1 and 5, such that a functional particle 23 is firmly attached to the portions except the band-like space portions 21a provided at a fixed interval on the second breathable base material 21 via the second adhesive agent 22. A width of the band-like space portion 21a is preferably set to about the same to between 1 or 2 times of a thickness of the functional particle carrying sheet. Specifically, the width of the band-like space portion 21a is preferably between 0.5 and 10 mm, and is more preferably between 0.7 and 5 mm, and is further preferable to be between 1 and 3 mm. If the width of the space portion is less than 0.5 mm, there is a case that it is hard to pleat the functional particle carrying sheet, or the functional particle jumps out of the breathable base material. Furthermore, if the width of the space portion gets over 10 mm, there is a case that the peak of the pleat is not precisely formed, the fluid excessively passes in the portion corresponding to the space portion, and the function of the functional particle is not sufficiently achieved.

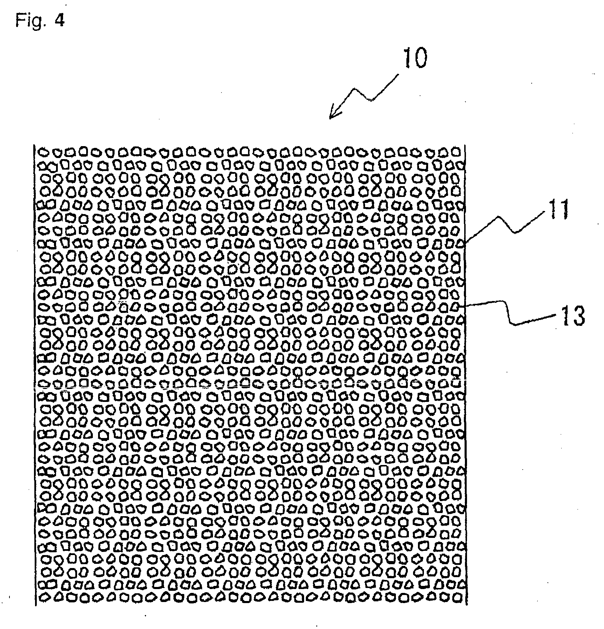

Furthermore, in the present invention, the first particle firmly attaching sheet 10 is structured, as shown in Figs. 1 and 4, such that the functional particle 13 is firmly attached onto the surface of the first breathable base material 11 via the first adhesive agent 12. In this case, in the first particle firmly attaching sheet 10, the functional particle 13 is firmly attached in a state in which the space portion 21a is not provided different from in the second particle firmly attaching sheet 20 mentioned above. As an aspect that the functional particle 13 is firmly attached, it is preferable to firmly attach the functional particle 13 in a state in which the interval between the functional particles 13 is wide, or wide space is provided, as far as the function of the functional particle is not lowered greatly. Specifically, it is preferable that the functional particle 13 is firmly attached at a rate between 20 and 99 % with respect to the maximum firmly attaching amount, it is more preferable that the functional particle 13 is firmly attached at a rate between 30 and 95 %, and it is further preferable that the functional particle 13 is firmly attached at a rate between 40 and 90 %. The functional particle carrying sheet exemplified by Fig. 2 shows an example in the case that the firmly attaching rate of the functional particle is lower than Fig. 1, in the first particle firmly attaching sheet 10. As mentioned above, since the functional particle 13 is firmly attached at 20 to 99 % with respect to the maximum firmly attaching amount, the portion of the breathable base material 11 positioned between the functional particles tends to be elongated or tends to be deformed. Accordingly, it is easy to pleat the functional particle carrying sheet. In this case, if the firmly attaching rate gets over 99 % with respect to the maximum firmly attaching amount, there is a case that it is hard to pleat the functional particle carrying sheet in some aspect of the functional particle. Furthermore, if the firmly attaching rate is less than 20 % with respect to the maximum firmly attaching amount, an amount of the functional particle is reduced too much. Therefore, the functionof the functional particle carrying sheet is lowered, or the fluid excessively passes in the peak portion of the pleat, so that there is a case that the function of the functional particle can not be sufficiently achieved. In this case, specifically, in the case that the functional particle is constituted by the gas removing particle mentioned above, it is preferable that the mass per unit area be between 50 and 500 g/m² , it is more preferable that the mass per unit area be between 70 and 400 g/m² , and it is further preferable that the mass per unit area be between 90 and 350 g/m².

In this case, the maximum firmly attaching amount means an amount at which the functional particle 13 can be firmly attached to the surface of the first breathable base material 11 to the maximum. In the actual measurement, a surplus functional particle is removed after scattering the functional particle on a flat plate, for example, to which an adhesive material is applied, and fixing the functional parti cle onto the flat plate. Next, a photography is carried out from above the flat plate to which the functional particle is attached, and an area rate A of the functional particle in the photographed image is calculated. On the other hand, a photography is carried out from a side to which the particle is firmly attached in the particle firmly attaching sheet corresponding to a comparative example and the area rate B of the functional particle in the photographed image is calculated. Thereafter, a rate R (%) with respect to the maximum fixing amount can be obtained by calculating an expression R (%) = 100 x B/A.

As mentioned above, in the first particle firmly attaching sheet 10, it is preferable that the functional particle 13 is firmly attached at a rate between 20 and 99 % with respect to the maximum firmly attaching amount. On the other hand, in the second particle firmly attaching sheet 20, it is preferable to firmly attach in a state in which the interval between the functional particles 23 is narrow, or in a state in which the space is provided less. Specifically, it is preferable that the functional particle 23 is firmly attached as much as possible, it is preferable that the functional particle 23 is firmly attached at 80 to 100 o with respect to the maximum firmly attaching amount in the portion except the band-like space portion 21a, and it is more preferable that it is firmly attached at 95 to 100 %. In this case, specifically, the functional particle is constituted by the gas removing particle mentioned above, and it is preferable that the mass per unit area be between 70 and 500 g/m², it is more preferable that the mass per unit area be between 90 and 400 g/m², and it is further preferable that the mass per unit area be between 110 and 350 g/m² . Furthermore, it is preferable that the interval between the functional particles 13 firmly attached to the first particle firmly attaching sheet 10 is relatively greater than the interval between the functional particles 23 firmly attached to the second particle firmly attaching sheet 20. Specifically, it is preferable to set the rate at which the functional particle is firmly attached onto the surface of the first breathable base material with respect to the maximum firmly attaching amount being smaller than the rate at which the functional particle firmly attached onto the surface of the portion except the band-like space portion of the breathable base material with respect to the maximum firmly attaching amount. In accordance with the aspect mentioned above, in the band-like space portion of the second particle firmly attaching sheet, the second breathable base material 21 is easy to elongate or deform, and the first breathable base material 11 is easy to be elongate or deform because the interval between the functional particles 13 is wide. Accordingly, it becomes even easier to pleat the functional particle carrying sheet, and it is possible to form an even sharper shape in the peak portion and the trough portion of the pleated shape. Furthermore, since it is possible to enlarge the firmly attaching rate with respect to the functional particle in the portion except the space portion of the second particle firmly attaching sheet 20, it is possible to sufficiently achieve the function of the functional particle.

In the present embodiment, the first adhesive agent 12 or the second adhesive agent 22 (hereinafter, the first adhesive agent and the second adhesive agent may be collectively referred simply to as an adhesive agent) is attached or applied onto the surface of the breathable base material 11 (or 21), however, in an attaching amount of the adhesive agent 12 (or 22), it is preferable that the mass per unit area be between 5 and 50 g/m², and it is further preferable that the mass per unit area be between 5 and 40 g/m² , in the area to which the adhesive agent is attached or applied. In this case, it is more preferable that the second adhesive agent 22 is attached or applied to the portion except the band-like space portions provided at the fixed interval on the surface of the second breathable base material 21. Accordingly, there is obtained an advantage that it is possible to save the adhesive agent and it is possible to securely obtain the space portion. As the adhesive agent 12 (or 22), the adhesive agent is not particularly limited as far as the functional particle can be firmly attached onto the surface of the base material, and there can be listed up a thermoplastic resin, a thermosetting resin, a moisture curing resin and the like.

If the adhesive agent is constituted by the thermoplastic resin, it is possible to firmly attach the functional particle by heating and melting. For example, as is different from the adhesive agent which is an emulsion of the thermosetting resin, a drying step is not necessary and it is possible to simplify the step. Accordingly, this structure is preferable. Furthermore, since there is no problem generated that the other additives than the resin are mixed, this structure is preferable. As the thermoplastic resin as mentioned above, can be used a thermoplastic polyamide resin, a thermoplastic polyester resin, a thermoplastic polyurethane resin, a polyolefin resin, a polyolefin modified resin and the like simply or in a mixed manner. The polyolefin modified resin mentioned here, can be listed up as an ethylene-vinyl acetate copolymer, a saponified resin of the ethylene-vinyl acetate copolymer, an ethylene-ethyl acrilate copolymer, an ethylene-acrylic acid copolymer, an ethylene-methacrylic acid copolymer, an ethylene-maleic acid copolymer, an ionomer resin (a heat sensitive resin obtained by adding a metal to the ethylene-methacrylic acid copolymer) and the like.

Furthermore, if the adhesive agent is constituted by the moisture curing resin, it is not necessary to heat for hardening the resin, but the resin can be hardened only by being exposed to an atmospheric air, so that the step can be simplified and this case is preferable. As the moisture curing resin mentioned above, for example, there is a polyurethane resin and the like, and there is a polyurethane resin having both of the moisture curing characteristic and thermal plasticity, and a polyurethane resin hardened by the moisture after being dissolved in a solvent. The polyurethane resin having both the natures of the moisture curing characteristic and the thermal plasticity is preferable, since the resin can be securely harden only by melting the resin so as to apply to the base material and subsequently exposing to the atmospheric air as it is.

As a preferable aspect that the first adhesive agent 12 is attached or applied onto the surface of the first breathable base material 11, there is an aspect of being obtained by a method of applying an adhesive agent in an emulsion state, an adhesive agent in a paste state or an adhesive agent in a thermal melting state to the base material by using a gravure roll. Further, there is an aspect obtained by a method of intermittently coating hot melt resin by a lot of nozzles arranged in one line, while using Porous Coat ^{™} of Nordson K.K. Further, there is an aspect of being obtained by a method of applying the adhesive agent in accordance with a spray method. Further, there is an aspect of being obtained by a method of scattering an adhesive agent in a particle state constituted by the thermal melting type resin onto the base material. Further, there is an aspect of being obtained by a method of heating the thermoplastic resin so as to set in a molten state, injecting the resin from the nozzle or the like, applying the resin to the base material or depositing the resin on a support body sheet having a mold release characteristic, thereby forming a hot melt nonwoven fabric formed as a webbed shape, and mounting the hot melt nonwoven fabric to the base material. In this case, in an aspect that the adhesive layer formed by the first adhesive agent has a breathability, it is possible to effectively use the functional particle carrying sheet in accordance with the present invention as the filter material.

Next, a description will be given of an aspect that the second adhesive agent 22 is attached or applied onto the surface of the second breathable base material 21. In the present invention, the second adhesive agent may be attached or applied onto the surface of the second breathable base material 21 without being provided with the band-like space portions 21a having the fixed interval, and the second functional particle 23 may be thereafter firmly attached to the portion except the band-like space portion provided at the fixed interval, however, for the purpose, it is necessary to bring the second functional particle 23 into contact at the fixed interval in accordance with a dispersion or the like, and there is a case that it is hard to manufacture. Accordingly, it is more preferable that the second adhesive agent 22 is attached or applied to the portion except the band-like space portions provided at the fixed interval on the surface of the second breathable base material 21, so that there is obtained an advantage that it is possible to securely obtain the space portion as well as the adhesive agent is saved.

As a preferable aspect that the second adhesive agent is applied such that the band-like space portion are provided at the fixed interval, the structure is not particularly limited as far as the band-like space portions are provided at the fixed interval, for example, there is an aspect obtained by a method of applying the adhesive agent in the emulsion state, the adhesive agent in the paste state, the adhesive agent in the thermal melding state or the like to the base material, for example, by using a gravure roll provided with a groove in a portion corresponding to the band-like space portion. Furthermore, in the method of intermittently coating the hot melt resin by a lot of nozzles arranged in one line using Porous Coat^{™} of Nordson K.K., there is an aspect obtained by a method of setting the space portions at a fixed interval by stopping the coating from each of the nozzles at a fixed interval. Furthermore, an aspect is obtained by method of applying the adhesive agent in accordance with the spray method such that the space portion comes to the fixed interval. Furthermore, there is an aspect obtained by a method of scattering the adhesive agent in the particle shape made of the thermal melting type resin onto the base material such that the space portion comes to the fixed gap. Furthermore, an aspect is obtained by method of heating the thermoplastic resin so as to set it in a molten state, ejecting out from the nozzle or the like, and applying to the base material such that the space portion comes to the fixed interval, or depositing it on the support body sheet having the mold releasing characteristic so as to form the hot melt nonwoven fabric having the webbed shape, and mounting the hot melt nonwoven fabric to the base material such that the space portion comes to the fixed interval. In this case, if an aspect is employed that the adhesive layer formed by the second adhesive agent has the breathability as an aspect that the second adhesive agent is applied such that the space portions are provided at the fixed interval, it is possible to effectively use the functional particle carrying sheet as the filter material.

As the thermoplastic resin which can be utilized in the hot melt nonwoven fabric, it is preferable to select the thermoplastic resin in which MI is equal to or more than 50 and equal to or less than 500. In the resin having MI which is lower than the preferable range, a fluidity is low at a time of heating process, and there is a case that the functional particle is incompletely attached firmly at a time of the thermal process. Further, in the resin having MI which is higher than the range mentioned above, the fluidity at a time of the heating process is high, and there is a case that the functional particle is incompletely attached firmly.

Furthermore, as a preferable aspect that the adhesive agent 12 (or 22) is applied onto the surface of the breathable base material, there is an aspect that the adhesive agent 12 (or 22) is regularly or irregularly applied in a dotted manner. Furthermore, there is an aspect that the adhesive agent 12 (or 22) is regularly or irregularly applied in a linear manner. In order to apply it in the dotted manner or the linear manner as mentioned above, for example, the gravure roll or the like may be used. Furthermore, the adhesive agent may be regularly applied in the dotted manner or the straight line manner by printing the thermoplastic resin having the paste shape in the dotted manner, in accordance with the method of the screen printing. Furthermore, it is possible to intermittently coat the hot melt resin by a lot of nozzles arranged in one line using Porous Coat^{™} of Nordson K.K. As mentioned above, it is possible to obtain the filter material in which the breathability is improved, by applying it in the dotted manner or the straight line manner. Furthermore, it is possible to obtain the application of the uniform adhesive agent in the stable state.

In this case, as the aspect that the second adhesive agent is applied such that the band-like space portions are provided at the fixed interval, for example, there is a method of applying the second adhesive agent 22 with the gravure roll without curving in the portion corresponding to the space portion. Furthermore, there is a method of printing the thermoplastic resin having the paste shape in the dotted manner after partly plugging the screen such that the space portion comes to the fixed gap, for example, in accordance with the screen printing method. Furthermore, in the method of intermittently coating the hot melt resin by a lot of nozzles arranged in one line using Porous Coat^{™} of Nordson K.K., there is a method of setting the space portions at a fixed interval by stopping the coating by each of the nozzles at a fixed interval.

Furthermore, as a preferable aspect that the adhesive agent 12 (or 22) is applied in the dotted manner on the surface of the breathable base material 11 (or 21), it is preferable that the size of the dot is close to a size of the functional particle. It is possible to match each of the dots and each of the functional particles substantially one by one by setting the size mentioned above, and it becomes easy to freely adjust the interval between the functional particles or the firmly attaching density of the functional particle by adjusting the interval of the dots. For example, it is easy to adjust it such as to firmly attach the functional particle 13 in the first particle firmly attaching sheet 10 at the rate of 20 to 99 % with respect to the maximum firmly attaching amount, and on the other hand, firmlyattach the functional particle 23 in the second particle firmly attaching sheet 20 at the rate of 80 to 100 % with respect to the maximum firmly attaching amount. Furthermore, it becomes easy to adjust it in such a manner as to make the rate of the firmly attaching of the functional particle 13 firmly attached to the first particle firmly attaching sheet 10 smaller than the rate of the firmly attaching of the functional particle 23 firmly attached to the portion except the band-like space portion of the second particle firmly attaching sheet 20. In specific, on the assumption that the size of the dot is expressed by the diameter of a circle having the same area as the area of the dot, and the size of the functional particle is expressed by the average particle diameter, it is preferable that the size of the functional particle be 0.5 to 3 times of the size of the dot, and it is more preferable that the size of the functional particle be 0.7 to 1.5 times. Furthermore, in the size of the dot, it is preferable that the diameter be between 0.1 and 2 mm, and it is more preferable that the diameter be between 0.15 and 1.5 mm. Furthermore, it is preferable that the distance between the centers of the adjacent dots be between 0.2 and 8 mm, and it is more preferable that the distance be between 0.3 and 6 mm. It is preferable that the rate of the center distance with respect to the size of the dot be between 1.05 and 4 times, and it is more preferable that the rate be between 1.05 and 2 times. In this case, the distance between the centers of the dots employs an average value of the distances between the centers of the dots.

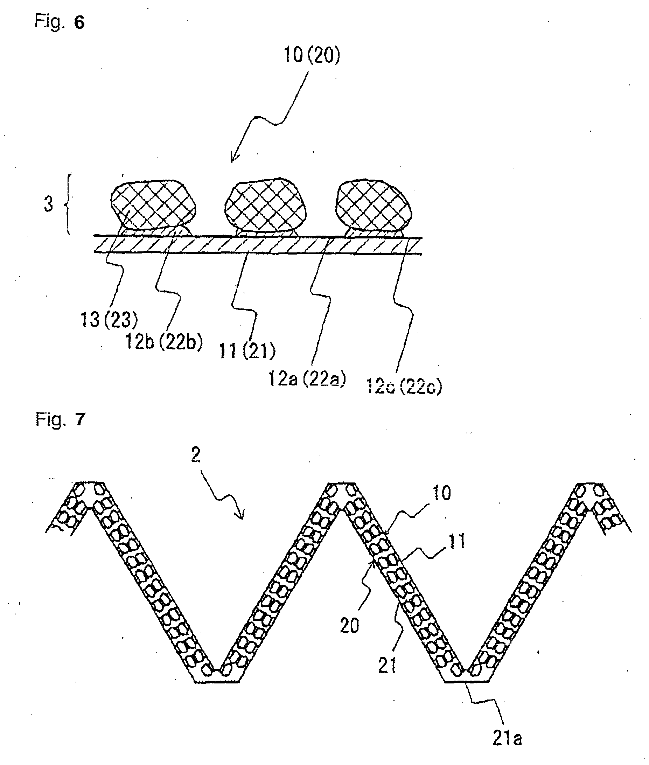

Furthermore, as the other preferable aspect that the adhesive agent 12 (or 22) is attached or applied onto the surface of the breathable base material 11 (or 21), for example, there is an aspect exemplified in Fig. 6. In Fig. 6, one surface of a functional particle layer 3 constituted by the functional particle 13 (or 23), and constituted by the adhesive agent 12a (or 22a) corresponding to a resin material (hereinafter, referred to as the "coupling portion") coupling the functional particle 13 (or 23) and the adhesive agent 12b (or 22b) corresponding to an aggregated resin material (hereinafter, referred to as the "resin aggregation portion") are firmly attached to the breathable base material 11 (or 21) by the adhesive agent 12b (or 22b).

As a method for obtaining the first particle firmly attaching sheet 10 or the second particle firmly attaching sheet 20 (hereinafter, the first particle firmly attaching sheet and the second particle firmly attaching sheet may be collectively referred simply to as a particle firmly attaching sheet) in accordance with the aspect mentioned above, it is possible to form the particle firmly attaching sheet 10 (or 20) in which one surface of the functional particle layer 3 is firmly attached to the breathable base material by the adhesive agent 12b (or 22b), by mounting a hot melt nonwoven fabric 12c (or 22c) on the breathable base material 11 (or 21) as shown in Fig. 6, thereafter arranging the functional particle 13 (or 23) in the surface of the hot melt nonwoven fabric 12c (or 22c), thereafter forming the resin aggregation portion 12b (or 22b) in the portion in which the hot melt nonwoven fabric and the functional particle 13 (or 23) come into contact in accordance with a heating process, forming a web constituted by the resin aggregation portion 12b (or 22b) and the coupling portion 12a (or 22a), and remaining only the functional particle firmly attached to the web in the functional particles.

In order to firmly attach the functional particle to the breathable base material, as mentioned above, it is possible to employ a method of applying the adhesive agent onto the surface of the breathable base material, thereafter bringing the functional particle into contact with the adhesive agent in accordance with the dispersion or the like, and subsequently heating and hardening or heating and melting the adhesive agent, and firmly attaching the functional particle to the base material, and further removing the surplus functional particle which is not firmly attached, as mentioned above. As mentioned above, it is possible to obtain the particle firmly attaching sheet to which the functional particle is firmly attachedvia the adhesive agent. Furthermore,intheparticlefirmly attachingsheetobtained as mentioned above, since the individual functional particle is firmly attached to the breathable base material via the adhesive agent in a part of the functional particle, an aspect is obtained that the functional particle is firmly attached at a thickness of one particle, and an aspect that the one layer of the functional particles are substantially firmly attached. Furthermore, it is preferable that a degree of the firmattachment is set to such a degree that the functional particle does not fall away from the breathable base material at a time of directing the particle firmly attaching sheet to a lower side in the functional particle side.

The functional particle carrying sheet in accordance with the present invention has the first particle firmly attaching sheet 10, and the second particle firmly attaching sheet 20. Since these two particle firmly attaching sheets have the band-like space portions 21a at a fixed interval only in the second particle firmly attaching sheet 20, a peak interval of the pleats is kept constant even if these two particle firmly attaching sheets are bonded. Accordingly, the first particle firmly attaching sheet 10 and the second particle firmly attaching sheet 20 may be identical or may be different in the portion except the portion of the space portion 21a. In other words, materials and shapes of the breathable base material, the adhesive agents, the functional particle or the like may be identical or may be different. For example, it is possible to achieve two kinds of functions by differentiating the functional particles firmly attached to the particle firmly attaching sheet from each other. Furthermore, it is possible to obtain a synergistic function of two kinds of functions.

The functional particle carrying sheet in accordance with the present invention has the first particle firmly attaching sheet 10, and the second particle firmly attaching sheet 20 structured such that the functional particle 23 is firmly attached to the portion except the band-like space portion 21a provided at a fixed interval, as exemplified in Figs. 1 and 2, and the functional particle 13 in the first particle firmly attaching sheet 10 and the functional particle 23 in the second particle firmly attaching sheet 20 are bonded via the third adhesive agent 30.

The third adhesive agent is not particularly limited as far as the third adhesive agent can bond the functional particle in the first particle firmly attaching sheet and the functional particle in the second particle firmly attaching sheet, and there can be listed up an adhesive agent constituted by a synthetic resin or the like. As the adhesive agent as mentioned above, a thermal adhesiveness resin is preferable, for example, a thermoplastic polyamide resin, a thermoplastic polyester resin, a thermoplastic polyurethane resin, a polyolefin resin, an ethylene-vinyl acetate copolymer resin or the like. Alternatively, a moisture curing type resin such as a polyurethane resin or the like is preferable. Furthermore, in the case of the polyurethane resin having both natures of the moisture curing characteristic and a thermal plasticity, it is possible to securely harden without necessity of heating, for example, by melting the resin, applying the resin to the functional particle of the particle firmly attaching sheet so as to bond to the functional particle in the other particle firmly attaching sheet, and subsequently exposing to the atmospheric air as it is.

In order to apply the thermal adhesiveness resin to the surface of the functional particle, for example, there is a method of heating the thermal adhesiveness resin so as to set to the molten state, injecting out of the nozzle or the like, and applying it to the surface of the functional particle in the webbed manner. Furthermore, it is possible to employ a method of heating the thermal adhesiveness resin so as to set to the molten state, injecting out of the nozzle or the like, depositing it on the mold release type support body sheet, separating a webbed hot melt nonwoven fabric 30a from the mold releasing type support body sheet, and arranging on the surface of the functional particles 13 and 23 as exemplified in Fig. 3. It is preferable that the attaching amount of the third adhesive agent as mentioned above be set to 5 to 50 g/m² in the mass per unit area, and it is further preferable that the attaching amount be set to 10 to 40 g/m² . In this case, at a time of applying the third adhesive agent such as the thermal adhesiveness resin or the like to the surface of the functional particle, in the case of an aspect that the adhesive layer formed by the third adhesive agent has the breathability, it is possible to effectively use the functional particle carrying sheet in accordance with the present invention as the filter material.

Next, a description will be given of a preferable example of the method of heating the thermal adhesiveness resin so as to set it to the molten state, ejecting out of the nozzle or the like, and applying it to the surface of the functional particle in the webbed manner. In other words , in the present invention, as the method of bonding via the third adhesive agent 30, as exemplified in Fig. 9, there is a method of simultaneously applying the hot melt resin 31 constituted by the third adhesive agent 30 to the surface of the functional particle in the first particle firmly attaching sheet 10 and the surface of the functional particle in the second particle firmly attaching sheet 20, and next overlapping and bonding the first particle firmly attaching sheet 10 and the second particle firmly attaching sheet 20. In accordance with this example, since it is possible to firmly bond the functional particles even if the applying amount of the third adhesive agent is small, there is an advantage that it is possible to manufacture the functional particle carrying sheet having the reduced pressure loss. Furthermore, there is an advantage that the cost can be reduced.

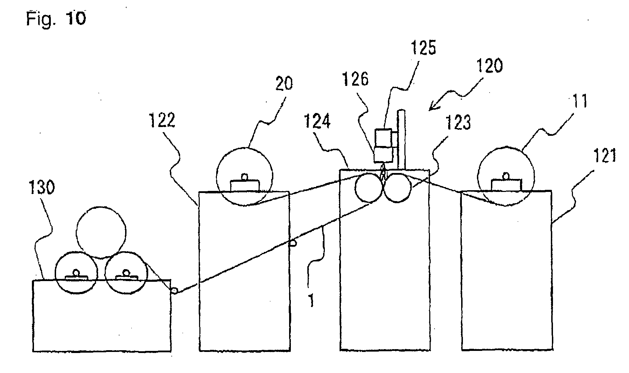

An apparatus shown in Figs. 9 and 10 is an apparatus which is preferably used for manufacturing of the functional particle carrying sheet in accordance with the present invention, and corresponds to a laminated integration sheet manufacturing apparatus 120 constituted by two sheet feed means 121 and 122 independently feeding out the first particle firmly attaching sheet 10 and the second particle firmly attaching sheet 20, a pair of rolls 123 continuously laminating two functional particle carrying sheets fed out by the sheet feed means while pinching, and a hot melt spray apparatus 125 arranged so as to head for a mating face of melt pair of rolls. The hot melt spray apparatus 125 can hot melt spray the hot melt resin 31 toward a boundary line formed by overlapping these two sheet-like materials, and is arranged in such a manner as to apply the hot melt resin to the mating surfaces of two sheet-like materials simultaneously.

A material of the hot melt resin is not particularly limited as far as the hot molt resin can be used as the hot melt adhesive agent, for example, there can be listed up an ethylene-vinyl acetate copolymer resin (EVA resin), a polyamide resin, a polyolefin copolymer resin, a polyester resin, an urethane resin, a reactive urethane resin, an elastomer resin, a synthetic rubber resin and the like. Furthermore, it is preferable that a softening point in accordance with "Testing methods of the softening point of hot melt adhesives" (JISK6863-1994) of the hot melt resin be between 70 and 180 °C, and it is more preferable that the softening point is between 80 and 160 °C. Further, it is preferable that a viscosity at a time of melting is low, for example, it is preferable that the viscosity is between 100 and 20000 mPa·s at 180°C, and it is more preferable that the viscosity is between 200 and 15000 mPa·s.

Furthermore, the hot melt resin is applied b y hot melt spray to the respective mating faces of two sheet-like materials simultaneously. In this case, the simultaneous application to the mating faces means that waiting times after the hot melt resin is applied until the mating faces are bonded in the respective mating faces of two sheet-like materials are identical in both the surfaces. It is preferable that the waiting time becomes shorter. Depending on the production speed and the position of the spray apparatus, it is preferable that the waiting time is equal to or less than 10 seconds, it is more preferable that the waiting time be equal to or less than 5 seconds, and it is further preferable that the waiting time be equal to or less than 1 second. For example, in the apparatus exemplified in Figs. 9 and 10, it is possible to make the waiting time equal to or less than 1 second.

Since the functional particle 13 in the first particle firmly attaching sheet 10 and the functional particle 23 in the second particle firmly attaching sheet 20 are bonded via the third adhesive agent 30 in accordance with the method as mentioned above, the following effects can be obtained. In other words, since it is possible to apply the hot melt resin to both the surfaces simultaneously by the hot melt spray and it is possible to extremely shorten the waiting time after the hot melt resin is applied until both the surfaces are bonded, it is possible to easily laminate and integrate two particle firmly attaching sheets in a state in which the surface of the hot melt resin tending to be cooled is still half molten, and it is possible to laminate and integrate firmly by a 1 ess amount of resin. For example, it is possible to set the mass per unit area between 1 and 60 g/m² , it is more preferable to set the mass per unit area between 3 and 50 g/m², and it is further preferable to set the mass per unit area between 5 and 40 g/m². Furthermore, even if the surface of the hot melt resin is cooled in some degree, the softening point is low and an excellent bonding property is provided in the hot melt resins. Accordingly, it is possible to firmly adhere to each other of the hot melt resin on the basis of a pressurizing. Furthermore, since it is not necessary to heat the sheet-like material to the melting temperature of the hot melt resin in the manufacturing method, it is possible to firmly and easily laminate and integrate without lowering the function of the functional particle.

The functional particle carrying sheet in accordance with the present invention has the first particle firmly attaching sheet 10, and the second particle firmly attaching sheet 20 structured such that the functional particle 23 is firmly attached to the portion except the band-like space portions 21a provided at the fixed interval, as mentioned above, and the functional particle 13 in the first particle firmly attaching sheet 10 and the functional particle 23 in the second particle firmly attaching sheet 20 are bonded via the third adhesive agent 30. Accordingly, if the functional particle carrying sheet 1 is pleated in the zigzag manner such that the band-like space portion 21a forms the peak or the trough of the pleat, as exemplified in Fig. 7, and is further formed, for example, as the functional particle carrying element 2 which is preferable as the filter element, as exemplified in Fig. 8, it is possible to obtain a sharp shape in the peak portion or the trough portion of the pleated shape. In other words, since the functional particles form one layer in the band-like space portion, and each of the particles is individually firmly attached to the base material, the individual particles follow the base material even if the base material is bent, and does not peel off from the base material. Furthermore, the portion of the breathable base material 11 (or 21) positioned between the functional particles tends to be elongated or deformed. Furthermore, the problem that the functional particle jumps out from the base material carrying the functional particle is not generated. Furthermore, it is possible to provide the functional particle carrying element in which the heights of the fold peaks are aligned precisely and have good quality, and the manufacturing apparatus of the element does not require the apparatus demanding the high technique, so that the manufacturing cost can be reduced.

Furthermore, in the functional particle carrying sheet in accordance with the present invention, since the functional particles are bonded to each other via the third adhesive agent 30, the problem is not generated in which the functional particles are closed up and the portion which does not come into contact with the processing fluid is generated. Therefore, it is possible to reduce the pressure loss without obstructing the original function of the functional particle, in spite that the function of the functional particle is increased.

In this case, the functional particle carrying element can be formed by pleating the functional particle carrying sheet in accordance with the present invention in the zigzag manner, and further bonding the frame material. For example, as exemplified in Fig. 8, the functional particle carrying element 2 can be obtained by applying the pleated processes at a predetermined pitch to the functional particle carrying sheet 1, bonding and fixing frame materials 40a and 40b made of a well-known material such as various synthetic resin, a paper, a metal material or the like for keeping the peak interval in correspondence to the design. In this case, Fig. 8 only shows a most general shape of the functional particle carrying element, and a shape constructing a fluid passing surface can be formed as a shaped corresponding to a device to which the functional particle carrying element is installed, such as a circular shape, a triangular shape, an oval shape and the like, in place of the exemplified rectangular shape.

In the case that the functional particle carrying element is used as a filter element for an intended use of a motor vehicle, for example, by setting the functional particle as a solid particle having a gas removing function, it is preferable to set a thickness of the functional particle carrying sheet to 0.2 to 4 mm, it is more preferable to set the thickness to 0.3 to 3 mm, and it is further preferable to set the thickness to 0.5 to 2 mm. In this case, the thickness means a thickness at a time of pressurizing at 0 .5 g/cm². Furthermore, it is preferable that the mass per unit area of the functional particle carrying sheet is between 150 and 1200 g/m² , it is more preferable that the mass per unit area be between 200 and 1000 g/m² , and it is further preferable that the mass per unit area be between 250 and 900 g/m². Furthermore, in a dimension of the functional particle carrying element, it is preferable that the height "h" be between 50 and 300 mm, it is preferable that the width "w" be between 50 and 300 mm, and it is preferable that a pleat depth "d" be between 15 and 300 mm. Furthermore, it is preferable that the pressure loss have a face velocity 3 . 0 m/sec of the functional particle carrying element is equal to or less than 200 Pa, and it is further preferable that the pressure loss is equal to or less than 150 Pa.

A description will be given below of the embodiments in accordance with the present invention, however, the embodiments correspond only to a preferable example for easily understanding the invention, and the present invention is not limited to contents of the embodiments.

### Embodiment

### (preparation of breathable base material)

A polyester spun bond partly thermally bonded and having a mass per unit area 30 g/m² is prepared as the first breathable base material 11 and the second breathable base material 21.

### (preparation of gravure roll A1)

A gravure roll A1 is prepared having a diameter of 150 mm and a width of 300 mm, having circular concave portions of a diameter of 0.4 mm and a depth of 0.1 mm on a surface thereof, and the concave portions being arranged on a straight line forming an angle of ± 45 degree with respect to the width direction of the roll such that an interval of the centers of the concave portions comes to 0.5 mm.

### (preparation of gravure roll A2)

A gravure roll A2 is prepared having a diameter of 150 mm and a width 300 mm, having circular concave portions of a diameter of 0.4 mm and a depth of 0.1 mm on the surface thereof, and the concave portions being arranged on a straight line forming an angle ± 45 degree with respect to a width direction of the roll such that an interval of the centers of the concave portions comes to 0.7 mm.

### (preparation of gravure roll B)

A gravure roll B is prepared having a diameter of 150 mm and a width of 300 mm, having circular concave portions of a diameter of 0.4 mm and a depth of 0.1 mm on a surface thereof, the concave portions being arranged on a straight line forming an angle of ± 45 degree with respect to the width direction of the roll such that an interval of the centers of the concave portions comes to 0.5 mm, band-like portions with a width 2.0 mm being provided at sixteen positions at a uniform interval over a whole in the width direction of the roll, and the band-like space portion being provided with no concave portion.

### (embodiment 1)

A moisture curing thermoplastic polyurethane resin is heated at 120 °C so as to be melted, and is attached to a surface having the concave portion of the prepared gravure roll A1, the gravure roll A1 is rotated, the prepared first base material 11 made of a spun bond is inserted to the gravure roll, and the moisture curing polyurethane resin is applied onto the base material in a dotted manner in such a manner that the mass per unit area comes to 15 g/m² . Subsequently, an activated carbon particle is applied to the base material by scattering the activated carbon particle in which an average particle diameter is 400 µm (95 % or more is distributed in a width of the particle diameter between 250 and 500 µm), from the above of the base material, during the molten state of the moisture hardening type polyurethane resin. Next, the first particle firmly attaching sheet 10 is obtained by cooling at a room temperature as it is, removing the surplus activated carbon particle which is not attached, and firmly attaching the activated carbon particle having the mass per unit area 130 g/m² to the base material. The particle firmly attaching sheet 10 has the shape shown in Fig. 4. Furthermore, a firmly attaching amount of the activated carbon particle is 100 % with respect to the maximum firmly attaching amount.

Next, in the same manner as mentioned above, the moisture curing thermoplastic polyurethane resin is heated at 120 °C so as to be melted, and is attached to a surface having the concave portion of the prepared gravure roll B, the gravure roll B is rotated, the prepared second base material 21 made of the spun bond is inserted to the gravure roll, and the moisture curing polyurethane resin is applied onto the base material in a dotted manner in such a manner that the mass per unit area comes to 14 g/m² . Subsequently, the activated carbon particle is applied to the base material by scattering the activated carbon particle in which an average particle diameter is 400 µm, from the above of the base material, during the molten state of the moisture curing polyurethane resin. Next, the second particle firmly attaching sheet 20 is obtained by cooling at a room temperature as it is, removing the surplus activated carbon particle which is not attached, and firmly attaching the activated carbon particle having the mass per unit area of 130 g/m² in the portion except the band-like space portion to the base material. The particle firmly attaching sheet 20 has the shape shown in Fig. 5. Further, a firmly attaching amount of the activated carbon particle is 100 % with respect to the maximum firmly attaching amount.

Next, a hot melt resin (a softening point 150 °C) made of a polyolefin copolymer is heated and melted. Next, the apparatus shown in Figs. 9 and 10 is prepared. Next, the heated and melted hot melt resin 31 mentioned above is sprayed to the surface of the functional particle in the first particle firmly attaching sheet 10 and the surface of the functional particle in the second particle firmly attaching sheet 20 from a nozzle 126, and simultaneously the hot melt resin 31 is applied to both the surfaces such that the mass per unit area comes to 20 g/m², and the first particle firmly attaching sheet 10 and the second particle firmly attaching sheet 20 are next overlapped and then bonded between a pair of rolls 123. Thereafter, the functional particle carrying sheet having the activated carbon particle of the mass per unit area 260 g/m² in the portion except the band-like space portion is obtained by cooling at room temperature and leaving it in the atmospheric air for a while.

### (embodiment 2)

A moisture curing thermoplastic polyurethane resin is heated at 120 °C so as to be molten, and is attached to a surface having the concave portion of the prepared gravure roll A2, the gravure roll A2 is rotated, the prepared first base material 11 made of a spun bond is inserted to the gravure roll, and the moisture curing polyurethane resin is applied onto the base material in a dotted manner in such a manner that the mass per unit area comes to 8 g/m². Subsequently, an activated carbon particle is applied to the base material by scattering the activated carbon particle in which an average particle diameter is 400 µm (95 % or more is distributed in a width of the particle diameter between 250 and 500 µm), from the above of the base material, during the molten state of the moisture curing polyurethane resin. Next, the first particle firmly attaching sheet 10 is obtained by cooling at a room temperature as it is, removing the surplus activated carbon particle which is not attached, and firmly attaching the activated carbon particle having the mass per unit area 105 g/m² to the base material. The particle firmly attaching sheet 10 has the shape shown in Fig. 4. Furthermore, a firmly attaching amount of the activated carbon particle of 81 % with respect to the maximum firmly attaching amount.

Thereafter step is set to the same as the embodiment 1, thereby obtaining the functional particle carrying sheet having the activated carbon particle of the mass per unit area 235 g/m² in the portion except band-like space portion.

### Description of Reference Numerals

- 1: functional particle carrying sheet
- 2: functional particle carrying element
- 3: functional particle layer
- 10: first particle firmly attaching sheet
- 11: first breathable base material
- 12: first adhesive agent
- 12a: coupling portion
- 12b: resin aggregation portion
- 12c: hot melt nonwoven fabric
- 13: first functional particle
- 20: second particle firmly attaching sheet
- 21: second breathable base material
- 21a: band-like space portion
- 22: second adhesive agent
- 22a: coupling portion
- 22b: resin aggregation portion
- 22c: hot melt nonwoven fabric
- 23: second functional particle
- 30: third adhesive agent
- 30a: hot melt nonwoven fabric
- 31: hot melt resin
- 40a: frame material
- 40b: frame material
- 41: separator
- 120: manufacturing apparatus of laminated integration sheet
- 121,: 122 sheet feed means
- 123: pair of rolls
- 125: hot melt spray apparatus
- 126: nozzle

## Claims

1. A functional particle carrying sheet (1) comprising:
a first particle firmly attaching sheet (10) structured such that functional particles (13) are firmly attached onto a surface of a first breathable base material (11) unexceptionally at a first firmly, attaching internal via a first adhesive agent (12); and
a second particle firmly attaching sheet (20) structured such that functional particles (23) are firmly attached onto a surface of a second breathable base material, (21) at a second firmly attaching interval except band-like space portions (21a) provided at a fixed interval via a second adhesive agent (22), wherein the functional particles (13) in the first particle firmly attaching sheet and the functional particles (23) in the second particle firmly attaching sheet (20) are bonded via a third adhesive agent (30).

2. A functional particle carrying sheet (1) as claimed in claim 1, wherein a width of said band-like space portions (21a) respectively is between 0.5 and 10 mm.

3. A functional particle carrying sheet (1) as claimed in claim 1 or 2, wherein an average particle diameter of said functional particles respectively is between 0.147 and 1.65 mm.

4. A functional particle carrying sheet (1) as claimed in any one of claims 1 to 3, wherein said functional particles (13) are firmly attached at 20 to 99% of a maximum firmly attaching amount onto the surface of said first breathable base material (11).

5. A functional particle carrying sheet (1) claimed in any one of claims 1 to 4, wherein the first firmly attaching interval between the functional particles (13) firmly attached to said first particle firmly attaching sheet is greater than the second firmly attaching interval between the functional particles (23) firmly attached to said second particle firmly attaching sheet (20).

6. A functional particle carrying sheet (1) claimed in any one of claims 1 to 5, wherein a rate at which said functional particles (13) are firmly attached onto the surface of said first breathable base material (11) with respect to the maximum firmly attaching amount is less than a rate at which said functional particles (23) are firmly attached onto the surface of the portion except the band-like space portions (21a) of said second breathable base material (20) with respect to the maximum firmly attaching amount.

7. A functional particle carrying sheet (1) as claimed in any one of claims 1 to 6, wherein the bonding via said third adhesive agent (30) is achieved by an application of a hot melt resin.

8. A functional particle carrying element, wherein the functional particle carrying sheet (1) as claimed in any one of claims 1 to 7 is pleated, and frame materials are firmly attached to a peripheral edge portion of said functional particle carrying sheet (1).

9. A manufacturing method of a functional particle carrying sheet (1) comprising the steps of:
preparing a first particle firmly attaching sheet (10) structured such that functional particles (13) are firmly attached onto a surface of a first breathable base material (11) unexceptionally at a first firmly attaching internal via a first adhesive agent (12), and a second particle firmly attaching sheet (20) structured such that functional particles (23) are firmly attached onto a surface of a second breathable base material (21) at a second firmly attaching interval, except band-like space portions provided at a fixed interval, via a second adhesive agent (22); and
next bonding the functional particles (13) in the first particle firmly attaching sheet (10) and the functional particles (23) in the second particle firmly attaching sheet (20) via a third adhesive agent (30).

10. A manufacturing method of a functional particle carrying sheet (1) as claimed in claim 9, wherein the method of bonding via said third adhesive agent (30) corresponds to a method of simultaneously applying a hot melt resin constituted by said third adhesive agent (30) to a surface of the functional particles (13) in the first particle firmly attaching sheet (10) and a surface of the functional particles (23) in the second particle firmly attaching sheet (20), and next overlapping and bonding the first particle firmly attaching sheet (10) and the second particle firmly attaching sheet (20).

## Patentansprüche

1. Ein Funktionspartikel-Trägerbogen (1), aufweisend:
einen ersten Partikel-Fest-Anbringungsbogen (10), der derart strukturiert ist, dass Funktionspartikel (13) ausnahmslos in einem ersten Fest-Anbringungs-Abstand über ein erstes Haftmittel (12) fest an einer Fläche eines ersten atmungsaktiven Basismaterials (11) angebracht sind,
einen zweiten Partikel-Fest-Anbringungsbogen (20), der derart strukturiert ist, dass Funktionspartikel (23) mit Ausnahme von bandartigen Abstandsabschnitten (21a), die in einem festen Abstand vorgesehen sind, in einem zweiten Fest-Anbringungs-Abstand über ein zweites Haftmittel (22) fest an einer Fläche eines zweiten atmungsaktiven Basismaterials (21) angebracht sind, wobei die Funktionspartikel (13) in dem ersten Partikel-Fest-Anbringungsbogen (10) und die Funktionspartikel (23) in dem zweiten Partikel-Fest-Anbringungsbogen (20) über ein drittes Haftmittel (30) miteinander verbunden sind.

2. Ein Funktionspartikel-Trägerbogen (1) gemäß Anspruch 1, wobei eine Breite der bandartigen Abstandsabschnitte (21a) jeweils zwischen 0,5 mm und 10 mm beträgt.

3. Ein Funktionspartikel-Trägerbogen (1) gemäß Anspruch 1 oder 2, wobei ein durchschnittlicher Partikeldurchmesser der Funktionspartikel jeweils zwischen 0,147 mm und 1,65 mm beträgt.

4. Ein Funktionspartikel-Trägerbogen (1) gemäß irgendeinem der Ansprüche 1 bis 3, wobei die Funktionspartikel (13) zu 20% bis 99% eines maximalen Fest-Anbringungsbetrags fest an der Fläche des ersten atmungsaktiven Basismaterials (11) angebracht sind.

5. Ein Funktionspartikel-Trägerbogen (1) gemäß irgendeinem der Ansprüche 1 bis 4, wobei der erste Fest-Anbringungs-Abstand zwischen den Funktionspartikeln (13), die fest an dem ersten Partikel-Fest-Anbringungsbogen angebracht sind, größer als der zweite Fest-Anbringungs-Abstand zwischen den Funktionspartikeln (23) ist, die fest an dem zweiten Partikel-Fest-Anbringungsbogen (20) angebracht sind.

6. Ein Funktionspartikel-Trägerbogen (1) gemäß irgendeinem der Ansprüche 1 bis 5, wobei eine Rate bezüglich des maximalen Fest-Anbringungs-Betrags, mit der die Funktionspartikel (13) fest an der Fläche des ersten atmungsaktiven Basismaterials (11) angebracht sind, kleiner als eine Rate bezüglich des maximalen Fest-Anbringungs-Betrags ist, mit der die Funktionspartikel (23) mit Ausnahme der bandartigen Abstandsabschnitte (21a) fest an der Fläche des Abschnitts des zweiten atmungsaktiven Basismaterials (20) angebracht sind.

7. Ein Funktionspartikel-Trägerboden (1) gemäß irgendeinem der Ansprüche 1 bis 6, wobei das Verbinden über das dritte Haftmittel (30) durch ein Aufbringen eines Heißschmelzharzes erreicht wird.

8. Ein Funktionspartikel-Trägerelement, wobei der Funktionspartikel-Trägerbogen (1) gemäß irgendeinem der Ansprüche 1 bis 7 gerafft ist und Rahmenmaterialien fest an einem Umfangsrandabschnitt des Funktionspartikel-Trägerbogens (1) angebracht sind.

9. Ein Herstellungsverfahren für einen Funktionspartikel-Trägerbogen (1), aufweisend die folgenden Schritte:
Vorbereiten eines ersten Partikel-Fest-Anbringungsbogens (10), der derart strukturiert ist, dass Funktionspartikel (13) über ein erstes Haftmittel (12) ausnahmslos in einem ersten Fest-Anbringungs-Abstand fest an einer Fläche eines ersten atmungsaktiven Basismaterials (11) angebracht sind, und eines zweiten Partikel-Fest-Anbringungs-Bogens (20), der derart strukturiert ist, dass Funktionspartikel (23) mit Ausnahme bandartiger Abstandsabschnitte, die in einem festen Abstand vorgesehen sind, über ein zweites Haftmittel (22) in einem zweiten Fest-Anbringungs-Abstand fest an einer Fläche eines zweiten atmungsaktiven Basismaterials (21) angebracht sind, und
anschließendes Verbinden der Funktionspartikel (13) in dem ersten Partikel-Fest-Anbringungs-Bogen (10) und der Funktionspartikel (23) in dem zweiten Partikel-Fest-Anbringungs-Bogen (20) über ein drittes Haftmittel (30).

10. Ein Herstellungsverfahren für einen Funktionspartikel-Trägerbogen (1) gemäß Anspruch 9, wobei das Verfahren des Verbindens über das dritte Haftmittel (30) einem Verfahren des gleichzeitigen Aufbringens eines Heißschmelzharzes, das durch das dritte Haftmittel (30) gebildet wird, auf eine Fläche der Funktionspartikel (13) in dem ersten Partikel-Fest-Anbringungs-Bogen (10) und eine Fläche der Funktionspartikel (23) in dem zweiten Partikel-Fest-Anbringungs-Bogen (20), und des anschließenden Überlappens und Verbindens des ersten Partikel-Fest-Anbringungs-Bogens (10) und des zweiten Partikel-Fest-Anbringungs-Bogens (20) entspricht.

## Revendications

1. Feuille portant des particules fonctionnelles (1) comprenant :
une première feuille de fixation ferme de particules (10) structurée de telle sorte que les particules fonctionnelles (13) soient fermement fixées sur une surface d'un premier matériau de base perméable à l'air (11) sans exception selon un premier intervalle de fixation ferme via un premier agent adhésif (12) ; et
une seconde feuille de fixation ferme de particules (20) structurée de telle sorte que les particules fonctionnelles (23) soient fermement fixées sur une surface d'un second matériau de base perméable à l'air (21) selon un second intervalle de fixation ferme à l'exception de parties d'espace similaires à une bande (21a) disposées selon un intervalle immuable via un deuxième agent adhésif (22), dans laquelle les particules fonctionnelles (13) dans la première feuille de fixation ferme de particules (10) et les particules fonctionnelles (23) dans la seconde feuille de fixation ferme de particules (20) sont collées via un troisième agent adhésif (30).

2. Feuille portant des particules fonctionnelles (1) selon la revendication 1, dans laquelle la largeur desdites parties d'espace similaires à une bande (21a) se situe respectivement entre 0,5 mm et 10 mm.

3. Feuille portant des particules fonctionnelles (1) selon la revendication 1 ou 2, dans laquelle le diamètre moyen de particule desdites particules fonctionnelles se situe respectivement entre 0,147 mm et 1,65 mm.

4. Feuille portant des particules fonctionnelles (1) selon l'une quelconque des revendications 1 à 3, dans laquelle lesdites particules fonctionnelles (13) sont fermement fixées à hauteur de entre 20 et 99 % d'une quantité de fixation ferme maximale sur la surface dudit premier matériau perméable à l'air (11).

5. Feuille portant des particules fonctionnelles (1) selon l'une quelconque des revendications 1 à 4, dans laquelle le premier intervalle de fixation ferme entre les particules fonctionnelles (13) fermement fixées sur ladite première feuille de fixation ferme de particules est plus grand que le second intervalle de fixation ferme entre les particules fonctionnelles (23) fermement fixées sur ladite seconde feuille de fixation ferme de particules (20).

6. Feuille portant des particules fonctionnelles (1) selon l'une quelconque des revendications 1 à 5, dans laquelle le taux auquel lesdites particules fonctionnelles (13) sont fermement fixées sur la surface dudit premier matériau perméable à l'air (11) par rapport à la quantité de fixation ferme maximale est inférieur au taux auquel lesdites particules fonctionnelles (23) sont fermement fixées sur la surface de la partie à l'exception des parties d'espace similaires à une bande (21a) dudit second matériau de base perméable à l'air (20) par rapport à la quantité de fixation ferme maximale.

7. Feuille portant des particules fonctionnelles (1) selon l'une quelconque des revendications 1 à 6, dans laquelle le collage via ledit troisième agent adhésif (30) est réalisé par l'application d'une résine thermofusible.

8. Élément portant des particules fonctionnelles, dans lequel la feuille portant des particules fonctionnelles (1) selon l'une quelconque de revendications 1 à 7 est plissée, et des matériaux d'encadrement sont fermement fixés sur une partie de bord périphérique de ladite feuille portant des particules fonctionnelles (1).

9. Procédé de fabrication d'une feuille portant des particules fonctionnelles (1) comprenant les étapes consistant à :
préparer une première feuille de fixation ferme de particules (10) structurée de telle sorte que les particules fonctionnelles (13) soient fermement fixées sur une surface d'un premier matériau de base perméable à l'air (11) sans exception selon un premier intervalle de fixation ferme via un premier agent adhésif (12), et une seconde feuille de fixation ferme de particules (20) structurée de telle sorte que les particules fonctionnelles (23) soient fermement fixées sur une surface d'un second matériau de base perméable à l'air (21) selon un second intervalle de fixation ferme à l'exception de parties d'espace similaires à une bande disposées selon un intervalle immuable via un deuxième agent adhésif (22) ; et
coller ensuite les particules fonctionnelles (13) dans la première feuille de fixation ferme de particules (10) et les particules fonctionnelles (23) dans la seconde feuille de fixation ferme de particules (20) via un troisième agent adhésif (30).

10. Procédé de fabrication d'une feuille portant des particules fonctionnelles (1) selon la revendication 9, dans lequel le procédé de collage via ledit troisième agent adhésif (30) correspond à un procédé consistant à appliquer simultanément une résine thermofusible constituée par ledit troisième agent adhésif (30) sur une surface des particules fonctionnelles (13) dans la première feuille de fixation ferme de particules (10) et sur une surface de particules fonctionnelles (23) dans la seconde feuille de fixation ferme de particules (20), et ensuite à superposer et coller la première feuille de fixation ferme de particules (10) et la seconde feuille de fixation ferme de particules (20).
